# EUROPEAN PATENT APPLICATION

(11) **EP 3 549 589 A1**
(43) Date of publication of application: **09.10.2019**
(21) Application number: 17877156.4
(22) Date of filing: 28.11.2017
(51) Int. Cl.: A61K 35/12, A61K 35/54, A61K 35/28, A61K 9/00

(54) **PHARMACEUTICAL COMPOSITION CONTAINING MITOCHONDRIA**

(30) Priority: 30.11.2016 KR 20160161059; 19.12.2016 KR 20160173748
(71) Applicant: Paean Biotechnology Inc., Daejeon 34028 (KR)
(72) Inventor: CHOI, Yong-Soo, Gunpo-si Gyeonggi-do 15888 (KR); YUN, Chang-koo, Hwaseong-si Gyeonggi-do 18472 (KR); KIM, Mi-Jin, Namyangju-si Gyeonggi-do 12178 (KR); HWANG, Jung Uk, Sejong 30082 (KR)
(74) Representative: Dehns
(86) International application number: PCT/KR2017/013707
(87) International publication number: WO 2018/101708

(57) **Abstract**

The present invention relates to a pharmaceutical composition containing mitochondria and, more specifically, to a pharmaceutical composition for preventing or treating muscle diseases or ischemic diseases, containing mitochondria as active ingredients. The pharmaceutical composition of the present invention contains heterologous mitochondria or cells comprising heterologous mitochondria, thereby enabling the mitochondrial activity of cells to which the same is administered to improve. Therefore, the pharmaceutical composition according to the present invention can be useful in the fundamental prevention or treatment of muscle diseases or ischemic diseases, which occur in relationship with the deterioration of mitochondrial function.

## Description

### Technical Field

The present invention relates to a pharmaceutical composition comprising mitochondria. More specifically, it pertains to a pharmaceutical composition for preventing or treating a muscle disease or an ischemic disease, comprising mitochondria as an active ingredient.

### Background Art

Mitochondria are organelles essential for the survival of eukaryotic cells, which are involved in the synthesis and regulation of adenosine triphosphate (ATP) used as an energy source. Mitochondria are associated with various metabolic pathways *in vivo,* such as cell signaling, cellular differentiation, and cell death, as well as the control of cell cycle and cell growth.

Thus, damage to mitochondria may lead to a variety of diseases, and most of the known mitochondrial disorders are caused by inherited or acquired mutations in mitochondrial DNA.

For example, the function of mitochondria may be altered by swelling due to abnormal mitochondrial membrane potential, by oxidative stress caused by reactive oxygen species and free radicals, etc., and by defects in the oxidative phosphorylation for energy production of mitochondria.

Specifically, mitochondria dysfunction has been known to be a direct or indirect cause of various diseases such as multiple sclerosis, encephalomyelitis, cerebral radiculoneuritis, peripheral neuropathy, Reye's syndrome, Alper syndrome, MELAS, migraine, psychosis, depression, seizures and dementia, paralytic episode, optic atrophy, optic neuropathy, retinal pigment degeneration, cataract, hyperaldosteronism, hypoparathyroidism, muscular disease, myoglobinuria, hypotonia, muscle pain, decrease in exercise tolerance, tubulopathy, renal failure, liver failure, hepatic dysfunction, liver hypertrophy, siderocyte anemia, neutropenia, thrombocytopenia, diarrhea, villus atrophy, multiple vomiting, dysphagia, constipation, sensorineural hearing loss, epilepsy, mental retardation, Alzheimer's disease, Parkinson's disease, and Huntington's disease (Schapira AH et al., Lancet., 1;368(9529):70-82, 2006; Pieczenik et al., Exp. Mol. Pathol., 83(1):84-92, 2007).

In particular, muscles require a high energy level and thus contain a relatively large amount of mitochondria, and mitochondria dysfunction may lead to a somewhat rapid progression of diseases in the muscles. For example, mitochondrial muscle diseases include MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, hypotonia, muscular weakness, myotonia, and the like.

In order to treat the diseases caused by mitochondria dysfunction, , studies have been conducted on materials that can be used to protect mitochondria or recover from mitochondrial dysfunction, or methods capable of efficiently delivering such substances to a target. However, no breakthrough therapeutics have yet been developed, due to the problems such as somewhat limited therapeutic range and occurrence of adverse side effects.

### Disclosure of Invention

### Technical Problem

An object of the present invention is to provide a pharmaceutical composition for treating a muscle disease and a method for treating a muscle disease using the same.

Another object of the present invention is to provide a pharmaceutical composition for treating an ischemic disease and a method for treating an ischemic disease using the same.

### Solution to Problem

In order to achieve the above object, the present invention provides a pharmaceutical composition for preventing or treating a muscle disease, comprising mitochondria as an active ingredient.

Also, the present invention provides a pharmaceutical composition for preventing or treating a muscle disease, comprising cells having exogenous mitochondria introduced therein as an active ingredient.

In addition, the present invention provides a method of preventing or treating a muscle disease, which comprises administering the pharmaceutical composition to the affected part of an animal.

Further, the present invention provides a pharmaceutical composition for preventing or treating an ischemic disease, comprising mitochondria as an active ingredient.

Also, the present invention provides a pharmaceutical composition for preventing or treating an ischemic disease, comprising cells having exogenous mitochondria introduced therein as an active ingredient.

In addition, the present invention provides a method of preventing or treating an ischemic disease, which comprises administering the pharmaceutical composition to the affected part of an animal via direct injection.

### Advantageous Effects of Invention

The pharmaceutical composition of the present invention comprises exogenous mitochondria or cells containing exogenous mitochondria as an active ingredient. Since the pharmaceutical composition of the present invention improves the mitochondrial activity of a subject to which it is administered, it can be effectively used for the fundamental prevention or treatment of a muscle disease caused by mitochondrial dysfunction.

### Brief Description of Drawings

Fig. 1 is a graph showing the change in body weight of rats wherein muscle atrophy is induced by administering dexamethasone.
Fig. 2 is a photograph showing the fluorescence staining of mitochondria in cells derived from rat skeletal muscle. At this time, the marker (Mitotracker CMXRos Red) is a dye of which accumulation is dependent on the mitochondrial membrane potential.
Fig. 3 is a graph showing the result of analyzing the proliferative capacity of cells after transferring exogenous mitochondria to muscle cells with impaired mitochondrial function.
Fig. 4 is a graph showing the result of analyzing the ATP synthesis capacity of cells after transferring exogenous mitochondria to muscle cells with impaired mitochondrial function.
Fig. 5 is a graph showing the result of analyzing the membrane potential of intracellular mitochondria after transferring exogenous mitochondria to muscle cells wherein muscle atrophy is induced.
Fig. 6 is a graph showing the result of analyzing mitochondrial reactive oxygen species after transferring exogenous mitochondria to muscle cells wherein muscle atrophy is induced.
Fig. 7 illustrates the result of analyzing the biosynthesis capacity of intracellular mitochondria after transferring exogenous mitochondria to muscle cells wherein muscle atrophy is induced.
Fig. 8a depicts the result of analyzing AMPK activity of cells after transferring exogenous mitochondria to muscle cells wherein muscle atrophy is induced.
Fig. 8b indicates the result of analyzing the amount of FoxO protein accumulated in cells after transferring exogenous mitochondria to muscle cells wherein muscle atrophy is induced.
Fig. 9 demonstrates the result of analyzing the expression level of MuRF-1, a muscle atrophy marker, after transferring exogenous mitochondria to muscle cells wherein muscle atrophy is induced.
Fig. 10 is a graph showing the weight changes of the soleus muscles after transferring exogenous mitochondria to rats wherein muscle atrophy is induced.
Fig. 11a displays the result of Western blot confirming the expression level of PGC-1, a protein involved in mitochondrial biosynthesis, after transferring exogenous mitochondria to rats wherein muscle atrophy is induced.
Fig. 11b is a graph showing the expression level of PGC-1, a protein involved in mitochondrial biosynthesis, after transferring exogenous mitochondria to rats wherein muscle atrophy is induced.
Fig. 12a presents the result of Western blot confirming the expression level of MuRF-1, a muscle atrophy marker, after transferring exogenous mitochondria to rats wherein muscle atrophy is induced.
Fig. 12b is a graph showing the expression level of MuRF-1, a muscle atrophy marker, after transferring exogenous mitochondria to rats wherein muscle atrophy is induced.
Fig. 13 is a photograph showing the result of histological examination confirming the regeneration process of myofibers following the transplantation of exogenous mitochondria.
Fig. 14 is photographs showing mitochondria of a target cell and a donor cell taken on a confocal scanning microscope.
Fig. 15 is a graph showing the results of visual evaluation of the therapeutic effect on critical limb ischemia using an animal model wherein critical limb ischemia is induced.
Fig. 16a illustrates the results of evaluating the therapeutic effect on critical limb ischemia through blood flow, using an animal model wherein critical limb ischemia is induced.
Fig. 16b depicts the results of evaluating the therapeutic effect on critical limb ischemia through blood flow, using an animal model wherein critical limb ischemia is induced.

### Best Mode for Carrying out the Invention

Hereinafter, the present invention will be described in detail.

In one aspect of the present invention, there is provided a pharmaceutical composition for preventing or treating a muscle disease, comprising mitochondria as an active ingredient.

Unless otherwise specified, the term "active ingredient" as used herein refers to an ingredient that exhibits activity alone or with an adjuvant (carrier) having no activity by itself.

The mitochondria may be those obtained from mammals and may be those obtained from humans. Specifically, the mitochondria may be those isolated from cells or tissues. The cell may be any one selected from the group consisting of somatic cells, germ cells, stem cells, and combinations thereof. For example, the mitochondria may be those obtained from somatic cells, germ cells, or stem cells. In addition, the mitochondria may be normal mitochondria obtained from cells whose mitochondrial biological activity is normal. In addition, the mitochondria may be those cultured *in vitro.*

Specifically, the somatic cell may be any one selected from the group consisting of muscle cells, hepatocytes, neurons, fibroblasts, epithelial cells, adipocytes, bone cells, leukocytes, lymphocytes, platelets, mucosal cells, and combinations thereof. Preferably, it may be the one obtained from muscle cells or hepatocytes excellent in mitochondrial activity.

In addition, the germ cell is a cell that undergoes meiosis and somatic cell division, and may be a sperm or an egg.

The stem cell may be any one selected from the group consisting of mesenchymal stem cells, adult stem cells, induced pluripotent stem cells, embryonic stem cells, bone marrow stem cells, neural stem cells, limbal stem cells, and tissue-derived stem cells. At this time, the mesenchymal stem cells may be those obtained from any one selected from the group consisting of umbilical cord, umbilical cord blood, bone marrow, fat, muscle, nerve, skin, amniotic membrane, and placenta.

On the other hand, when isolating the mitochondria from specific cells, it is possible to isolate the mitochondria by various known methods, for example, by using a specific buffer solution or using a potential difference and a magnetic field.

In terms of maintaining mitochondrial activity, the isolation of mitochondria may be performed by disrupting and centrifuging the cells. In one embodiment, it may be performed by the steps of: culturing the cells, subjecting a pharmaceutical composition comprising the cells to the first centrifugation to produce pellets, suspending and homogenizing the pellets in a buffer solution, subjecting the resulting homogenized solution to the second centrifugation to produce a supernatant, and subjecting the supernatant to the third centrifugation to purify mitochondria. At this time, it is preferable that the time for performing the second centrifugation is adjusted to be shorter than the time for performing the first and third centrifugations, in terms of maintaining cell activity, and the speed of centrifugation may be increased from the first centrifugation to the third centrifugation.

Specifically, the first to the third centrifugation may be performed at a temperature of 0 to 10 °C, preferably 3 to 5 °C. In addition, the time for performing the centrifugation may be 1 to 50 minutes, and may be appropriately adjusted according to the number of centrifugations and the amount of the sample.

In addition, the first centrifugation may be performed at a speed of 100 to 1,000 x g, 200 to 700 x g, or 300 to 450 x g. Further, the second centrifugation may be performed at a speed of 1 to 2,000 x g, 25 to 1,800 x g, or 500 to 1,600 x g. Further, the third centrifugation may be performed at a speed of 100 to 20,000 x g, 500 to 18,000 x g, or 800 to 15,000 x g.

In addition, the muscle disease to which the pharmaceutical composition can effectively be applied may be a disease including mitochondrial dysfunction. For example, the muscle disease may be MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, hypotonia, muscular weakness, or myotonia, and may include all muscle cell disorders caused by mitochondrial hypofunction.

That is, the pharmaceutical composition of the present invention includes mitochondria whose cellular activity is maintained, as an active ingredient, and thus can enhance the mitochondrial activity of cells to which the pharmaceutical composition is applied, thereby improving the symptoms of mitochondrial diseases.

In addition, for the above pharmaceutical composition, the mitochondria may be contained at a concentration of 0.1 to 500 µg/ml, 0.2 to 450 µg/ml, or 0.5 to 400 µg/ml. By including the mitochondria in the above range, the dose of the mitochondria can be easily controlled upon administration, and the degree of improvement of the muscle disease symptoms of the affected part can be further enhanced.

In particular, the pharmaceutical composition according to the present invention may include mitochondria so that 0.005 to 50 µg or 0.05 to 25 µg of mitochondria can be administered per 1 × 10⁵ cells on the basis of the cells to which mitochondria are to be delivered. That is, it is most preferable in terms of cellular activity that the mitochondria are administered in a content of the above range based on the number of cells to which the pharmaceutical composition is to be administered or the number of cells in the affected part caused by a muscle disease. In addition, the pharmaceutical composition may be administered at a dose of 1 to 80 µl, 10 to 70 µl, or 40 to 60 µl per administration.

In addition, another aspect of the present invention provides a pharmaceutical composition for preventing or treating a muscle disease, comprising cells with exogenous mitochondria introduced therein as an active ingredient. The exogenous mitochondria refer to mitochondria originated from different cells except the cells into which the mitochondria are to be introduced. Details of the exogenous mitochondria and the method for isolating them are as described above.

In addition, the cells may be cells containing exogenous mitochondria in the cytoplasm, and may be normal cells whose cellular activities are maintained. In addition, the cells are preferably muscle cells or stem cells. At this time, the muscle cells may be cells isolated from muscle tissues, or may be cells obtained by further culturing the isolated cells. Further, the stem cells may be any one selected from the group consisting of mesenchymal stem cells, adult stem cells, induced pluripotent stem cells, embryonic stem cells, bone marrow stem cells, neural stem cells, limbal stem cells, and tissue-derived stem cells. At this time, the mesenchymal stem cells may be obtained from any one selected from the group consisting of umbilical cord, umbilical cord blood, bone marrow, fat, muscle, nerve, skin, amniotic membrane, and placenta.

In addition, the cells into which the exogenous mitochondria are introduced and the cells that provide the exogenous mitochondria may be allogeneic or xenogeneic. In addition, the cells into which the exogenous mitochondria are introduced and the cells that provide the exogenous mitochondria may be obtained from the same subject or from different subjects.

Meanwhile, the pharmaceutical composition containing a mixture of the exogenous mitochondria and the cells to be delivered therewith may be centrifuged to deliver the exogenous mitochondria into the cells. In addition, the centrifugation may be carried out at a temperature of 0 to 40 °C, 20 to 38 °C, or 30 to 37 °C. It may also be carried out for 0.5 to 20 minutes, 1 to 15 minutes, or 3 to 7 minutes. Further, it may be carried out at 1 to 2,400 x g, 25 to 1,800 x g, or 200 to 700 x g.

By applying the centrifugal force simultaneously to the cells and the exogenous mitochondria under the above conditions, the exogenous mitochondria can be delivered to the cells with high efficiency without causing any damage to the cells.

In addition, it may be more efficient to centrifuge the pharmaceutical composition in a test tube having a gradually decreasing diameter toward the lower end thereof, in terms of the centrifugal force applied to the cells and the mitochondria, and the contact efficiency. The centrifugation may be performed one to three additional times under the same conditions.

In addition, before performing the centrifugation, a step of incubating the pharmaceutical composition may be further included. The incubation may be performed at a temperature of 0 to 40 °C, 20 to 38 °C, or 30 to 37 °C. Also, it may be carried out for 0.1 to 4 hours, 0.5 to 3.8 hours or 0.8 to 3.5 hours. In addition, the incubation may be performed for a predetermined time after the mitochondria are delivered to the cells. Further, the incubation time may be appropriately selected depending on the kind of cells and the amount of mitochondria.

In addition, a step of adding a surfactant to the pharmaceutical composition may be further included. Surfactants are used to enhance the cell membrane permeability of the cells, and the time of addition may be before, during, or after mixing the cells and the mitochondria. In addition, after the surfactant is added to the cells, the cells may be allowed to stand for a certain period of time in order to increase the cell membrane permeability. Specifically, a step of adding PF-68 (Pluronic F-68) to the pharmaceutical composition may be included.

Specifically, the surfactant is preferably a nonionic surfactant such as PF-68, and may be poloxamers, that is, nonionic triblock copolymers composed of a central hydrophobic chain of polyoxypropylene flanked by two hydrophilic chains of polyoxyethylene. In addition, the concentration of the surfactant in the pharmaceutical composition may be 1 to 100 µg/ml, 3 to 80 mg/ml, or 5 to 40 µg/ml, preferably 10 to 30 µg/ml.

In addition, the cells contained in the pharmaceutical composition may be those where 0.1 to 500 µg, 0.2 to 450 µg, or 0.5 to 400 µg of mitochondria are introduced per 1 x 10⁵ cells. The pharmaceutical composition contains the cells having exogenous mitochondria transferred thereto in such an amount, thereby can improve the cell function of the affected part of muscle disease upon administration thereof.

In the case of the pharmaceutical composition which comprises the cells received mitochondria as an active ingredient, the dosage may be 2 x 10³ to 2 x 10⁷ cells/kg (body weight), more preferably 1 x 10⁴ to 1 x 10⁷ cells/kg (body weight). It is effective in terms of dose control and improvement of muscle disease to contain cells having exogenous mitochondria introduced therein at an amount within the above range.

In addition, the muscle disease to which the pharmaceutical composition can effectively be applied may be a disease including mitochondrial dysfunction. For example, the muscle disease may be MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, hypotonia, muscular weakness, or myotonia, and may include all muscle cell disorders caused by mitochondrial hypofunction.

In addition, the pharmaceutical compositions according to the present invention may be administered to humans or other mammals who may get, or are suffering from, mitochondrial muscle disease or disorders. In addition, the pharmaceutical composition may be in the form of an injection that can be directly administered to the affected part, preferably a preparation for intramuscular injection.

Therefore, in order to ensure product stability during distribution of injection preparations, the pharmaceutical composition according to the present invention may be manufactured into an injection, which is physically or chemically very stable, by adjusting pH with an aqueous acid solution or a buffer such as a phosphate buffer which can be used for injection.

Specifically, the pharmaceutical composition of the present invention may contain water for injection. The water for injection is distilled water prepared for dissolving a solid injection or diluting an aqueous injection, and specifically, prepared for glucose injection, xylitol injection, D-mannitol injection, fructose injection, physiological saline, dextran 40 injection, dextran 70 injection, amino acid injection, Ringer's solution, lactic acid-Ringer's solution, or phosphate buffer or monobasic sodium phosphate-citrate buffer in the range of pH 3.5 to 7.5.

In addition, the pharmaceutical composition of the present invention may contain a stabilizer or a solubilizing agent. For example, the stabilizer may be sodium pyrosulfite or ethylenediaminetetraacetic acid, and the solubilizing agent may be hydrochloric acid, acetic acid, sodium hydroxide, sodium hydrogen carbonate, sodium carbonate, or potassium hydroxide.

Moreover, the present invention provides a method of preventing or treating a muscle disease, which comprises administering the above-mentioned pharmaceutical composition directly to the affected part of a subject. The subject may be a mammal, preferably a human.

So, the pharmaceutical composition according to the present invention can supply exogenous mitochondria having normal activity directly to the affected part in which muscle disease occurs, and thus is useful for increasing the activity of cells having mitochondrial hypofunction or for regenerating cells having mitochondrial dysfunction, and may be used for the treatment or prevention of muscle diseases caused by mitochondrial dysfunction.

Further, the present invention provides a use of the above pharmaceutical composition for preventing or treating a muscle disease.

Furthermore, the present invention provides a use of the above pharmaceutical composition for the manufacture of a medicament for preventing or treating a muscle disease.

Another aspect of the present invention provides a pharmaceutical composition for preventing or treating an ischemic disease, comprising mitochondria as an active ingredient.

Unless otherwise specified, the term "active ingredient" as used herein refers to an ingredient that exhibits activity alone or with an adjuvant (carrier) having no activity by itself.

The mitochondria may be those obtained from mammals and may be those obtained from humans. In addition, the mitochondria may be those isolated from cells or cell cultures. Specifically, the mitochondria may be those isolated from cells or tissues. The cell may be any one selected from the group consisting of somatic cells, germ cells, stem cells, and combinations thereof. For example, the mitochondria may be those obtained from somatic cells, germ cells, stem cells, or combinations thereof, which are derived from mammals or humans. In addition, the mitochondria may be normal mitochondria obtained from cells whose mitochondrial biological activity is normal. In addition, the mitochondria may be those cultured *in vitro.*

Specifically, the somatic cell may be any one selected from the group consisting of muscle cells, hepatocytes, neurons, fibroblasts, epithelial cells, adipocytes, bone cells, leukocytes, lymphocytes, platelets, mucosal cells, and combinations thereof. Preferably, they may be those obtained from muscle cells or hepatocytes excellent in mitochondrial activity.

In addition, the germ cell is a cell that undergoes meiosis and somatic cell division, and may be a sperm or an egg.

The stem cells may be any one selected from the group consisting of mesenchymal stem cells, adult stem cells, induced pluripotent stem cells, embryonic stem cells, bone marrow stem cells, neural stem cells, limbal stem cells, and tissue-derived stem cells. At this time, the mesenchymal stem cells may be those obtained from any one selected from the group consisting of umbilical cord, umbilical cord blood, bone marrow, fat, muscle, nerve, skin, amniotic membrane, and placenta.

On the other hand, when isolating the mitochondria from specific cells, it is possible to isolate the mitochondria by various known methods, for example, by using a specific buffer solution or using a potential difference and a magnetic field. The details of mitochondrial isolation are as described above.

In addition, ischemic diseases to which the above pharmaceutical composition can be effectively applied are diseases involving irreversible damage of the tissue-constituting cells caused by reduced supply of blood flow, wherein processes called ischemic cascades are triggered, resulting in permanent loss of the functions of brain, heart, or peripheral tissues. For example, the ischemic diseases may be critical limb ischemia, ischemic stroke, ischemic heart disease, or ischemic colitis. At this time, the ischemic diseases may be diseases caused by mitochondrial dysfunction, and may include all ischemic cell disorders caused by mitochondrial hypofunction.

That is, the pharmaceutical composition of the present invention includes mitochondria whose cellular activity is maintained, as an active ingredient, and thus can enhance the mitochondrial activity of cells to which the pharmaceutical composition is applied, thereby improving the symptoms of mitochondrial diseases.

In addition, for the above pharmaceutical composition, the mitochondria may be contained at a concentration of 0.1 to 500 µg/ml, 0.1 to 200 µg/ml, or 0.2 to 10 µg/ml. By including the mitochondria in the above range, the dose of the mitochondria can be easily controlled upon administration, and the degree of improvement of the ischemic disease symptoms of the affected part can be further enhanced.

In addition, another aspect of the present invention provides a pharmaceutical composition for preventing or treating an ischemic disease, comprising cells with exogenous mitochondria introduced therein as an active ingredient. The exogenous mitochondria refer to mitochondria originated from different normal cells except the cells into which the mitochondria are to be introduced. Details of the exogenous mitochondria and the method for isolating them are as described above.

In addition, the cells may be cells containing exogenous mitochondria in the cytoplasm, and may be normal cells whose cellular activities are maintained. In addition, the cells are preferably somatic cells, germ cells, stem cells, or combinations thereof. At this time, the cells may be isolated cells or may be cells obtained by further culturing the isolated cells. Further, the stem cells may be any one selected from the group consisting of mesenchymal stem cells, adult stem cells, induced pluripotent stem cells, embryonic stem cells, bone marrow stem cells, neural stem cells, limbal stem cells, and tissue-derived stem cells. At this time, the mesenchymal stem cells may be obtained from any one selected from the group consisting of umbilical cord, umbilical cord blood, bone marrow, fat, muscle, nerve, skin, amniotic membrane, and placenta.

In addition, the cells into which the exogenous mitochondria are introduced and the cells that provide the exogenous mitochondria may be allogeneic or xenogeneic. In addition, the cells into which the exogenous mitochondria are introduced and the cells that have provided the exogenous mitochondria may be obtained from the same subject or from different subjects.

In addition, the cells contained in the pharmaceutical composition may be those where 0.1 to 500 µg, 0.2 to 450 µg, or 1 to 300 µg of mitochondria are introduced per 1 x 10⁵ cells. The pharmaceutical composition contains the cells with exogenous mitochondria transferred thereto in such an amount, thereby can improve the cell function of the affected part of ischemic disease upon administration thereof.

Meanwhile, the pharmaceutical composition containing a mixture of the exogenous mitochondria and the cells to be delivered therewith may be centrifuged to deliver the exogenous mitochondria into the cells. In addition, the centrifugation may be carried out at a temperature of 0 to 40 °C, 20 to 38 °C, or 30 to 37 °C. It may also be carried out for 0.5 to 20 minutes, 1 to 15 minutes, or 3 to 7 minutes. Further, it may be carried out at 1 to 2,400 x g, 25 to 1,800 x g, or 200 to 700 x g.

By applying the centrifugal force simultaneously to the cells and the exogenous mitochondria under the above conditions, the exogenous mitochondria can be delivered to the cells with high efficiency without causing any damage to the cells.

In addition, it may be more efficient to centrifuge the pharmaceutical composition in a test tube having a gradually decreasing diameter toward the lower end thereof, in terms of the effective centrifugal force applied to the cells and the mitochondria, and the contact efficiency. The centrifugation may be performed one to three additional times under the same conditions.

In addition, before performing the centrifugation, a step of incubating the pharmaceutical composition may be further included. The incubation may be performed at a temperature of 0 to 40 °C, 20 to 38 °C, or 30 to 37 °C. Also, it may be carried out for 0.1 to 4 hours, 0.5 to 3.8 hours or 0.8 to 3.5 hours. In addition, the incubation may be performed for a predetermined time after the mitochondria are delivered to the cells. Further, the incubation time may be appropriately selected depending on the kind of cells and the amount of mitochondria.

In addition, a step of adding a surfactant to the pharmaceutical composition may be further included. Surfactants are used to enhance the cell membrane permeability of the cells, and the time of addition may be before, during, or after mixing the cells and the mitochondria. In addition, after the surfactant is added to the cells, the cells may be allowed to stand for a certain period of time in order to increase the cell membrane permeability. Specifically, a step of adding PF-68 (Pluronic F-68) to the pharmaceutical composition may be included.

Specifically, the surfactant is preferably a nonionic surfactant such as PF-68, and may be poloxamers, that is, nonionic triblock copolymers composed of a central hydrophobic chain of polyoxypropylene flanked by two hydrophilic chains of polyoxyethylene. In addition, the concentration of the surfactant in the pharmaceutical composition may be 1 to 100 mg/ml, 3 to 80 mg/ml, or 5 to 40 mg/ml, preferably 10 to 30 mg/ml.

In addition, ischemic diseases to which the above pharmaceutical composition can be effectively applied are diseases involving irreversible damage of the tissue-constituting cells caused by reduced supply of blood flow, wherein processes called ischemic cascades are triggered, resulting in permanent loss of the functions of brain, heart, or peripheral tissues. For example, the ischemic diseases may be critical limb ischemia, ischemic stroke, ischemic heart disease, or ischemic colitis. At this time, the ischemic diseases may be diseases caused by mitochondrial dysfunction, and may include all ischemic cell disorders caused by mitochondrial hypofunction.

In addition, the pharmaceutical compositions according to the present invention may be administered to humans or other mammals who may get, or are suffering from, mitochondrial ischemic disease or disorders. In addition, the pharmaceutical composition may be in the form of an injection that can be directly administered to the affected part, preferably a preparation for intramuscular injection.

Therefore, in order to ensure product stability during distribution of injection preparations, the pharmaceutical composition according to the present invention may be manufactured into an injection, which is physically or chemically very stable, by adjusting pH with an aqueous acid solution or a buffer such as a phosphate buffer which can be used for injection.

Specifically, the pharmaceutical composition of the present invention may contain water for injection. The water for injection is distilled water prepared for dissolving a solid injection or diluting an aqueous injection, and specifically, prepared for glucose injection, xylitol injection, D-mannitol injection, fructose injection, physiological saline, dextran 40 injection, dextran 70 injection, amino acid injection, Ringer's solution, lactic acid-Ringer's solution, or phosphate buffer or monobasic sodium phosphate-citrate buffer in the range of pH 3.5 to 7.5.

In addition, the pharmaceutical composition of the present invention may contain a stabilizer or a solubilizing agent. For example, the stabilizer may be sodium pyrosulfite or ethylenediaminetetraacetic acid, and the solubilizing agent may be hydrochloric acid, acetic acid, sodium hydroxide, sodium hydrogen carbonate, sodium carbonate, or potassium hydroxide.

Moreover, the present invention provides a method of preventing or treating an ischemic disease, which comprises administering the above-mentioned pharmaceutical composition directly to the affected part of a subject. The subject may be a mammal, preferably a human.

So, the pharmaceutical composition according to the present invention can supply exogenous mitochondria having normal activity directly to the affected part in which an ischemic disease occurs, and thus is useful for increasing the activity of cells having mitochondrial hypofunction or for regenerating cells having mitochondrial dysfunction, and may be used for the treatment or prevention of an ischemic disease caused by mitochondrial dysfunction.

Further, the present invention provides a use of the above pharmaceutical composition for preventing or treating an ischemic disease.

Furthermore, the present invention provides a use of the above pharmaceutical composition for the manufacture of a medicament for preventing or treating an ischemic disease.

### Mode for the Invention

Hereinafter, preferred embodiments of the present invention will be described in order to facilitate understanding of the present invention. However, the following examples are provided only for the purpose of easier understanding of the present invention, and the present invention is not limited by the following examples.

### I. Confirmation of the effects of treating a muscle disease of mitochondria and cells containing exogenous mitochondria

### Example 1. Establishment of a muscle atrophy animal model and measurement of weight change

Five-week-old female SD rats as experimental animals were purchased from Orient Bio Co., Ltd. (Seoul, Korea). The rats were acclimatized in the clean zone of the Experimental Animal Center of CHA University. During the acclimation period, the environment of the rats was maintained at a 12-h light/dark cycle, a room temperature of 23 ± 2 °C, and a humidity of 40 to 60%. After 7 days of such acclimation period, the rats were subjected to the experiments.

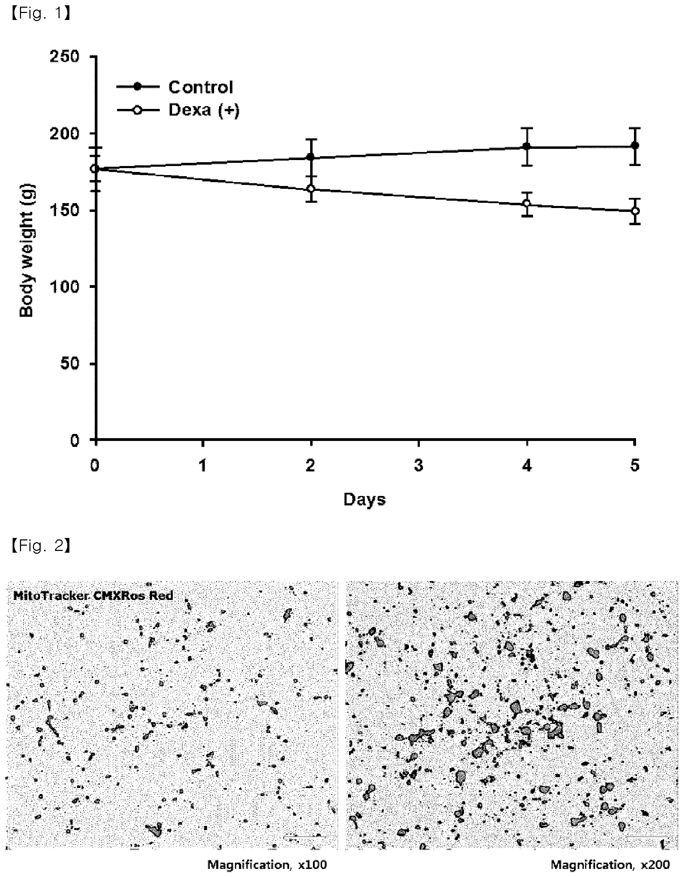

To induce muscle atrophy, dexamethasone was administered intraperitoneally to the rats at a dose of 5 mg/kg for 5 days. The rats were weighed daily, and it was confirmed that the weight of the dexamethasone-treated group was reduced by about 30% compared to the normal group on the 5th day of dexamethasone administration (Fig. 1).

### Example 2. Preparation of mitochondria for transplantation

As a donor cell of mitochondria, rat skeletal muscle-derived cell line L6 (CRL1458, ATCC, Manassas, VA, USA) was cultured and the resulting cells were stained with mitochondria-specific markers (Mitotracker CMXRos Red).

In order to extract mitochondria, the number of cells was measured using a hemocytometer to recover about 2 x 10⁷ cells/ml of cells. Then, the cells were subjected to the first centrifugation at a temperature of about 4 °C for 10 minutes at a speed of 350 × g, and the resulting pellet was recovered and resuspended in a buffer solution, followed by homogenization. The pharmaceutical composition containing the pellet was subjected to the second centrifugation at a temperature of about 4 °C for 3 minutes at a speed of 1,100 x g to obtain a supernatant. The supernatant was then subjected to the third centrifugation at a temperature of about 4 °C for 15 minutes at a speed of 12,000 x g to isolate the mitochondria from the cells.

In order to identify the isolated mitochondria, the intracellular mitochondria labeled with red fluorescence before the isolation was photographed by a fluorescence microscope and shown in Fig. 2, and isolated mitochondria were identified. In particular, the marker used at this time (Mitotracker CMXRos Red) is a dye of which accumulation is dependent on the mitochondrial membrane potential, showing the viability of isolated mitochondria (Fig. 2).

### Example 3. Evaluation of proliferative capacity of cells having received mitochondria

L6 cells, a rat skeletal muscle-derived cell line commonly used for cell-related experiments, were used as a control. In addition, L6 cells were treated with mitochondrial ATP synthesis inhibitor (oligomycin, Sigma-Aldrich, St. Louis, MO, USA) to artificially suppress mitochondrial function. Thereafter, healthy mitochondria extracted from WRL-68 hepatic cells (CL48, ATCC) were transferred to 1×10⁵ L6 cells at different concentrations (0.05, 0.5, and 5 µg), and the resulting cells were seeded into a 24-well plate and incubated at 37 °C in a CO₂ incubator.

In order to compare the proliferative capacity of cells, the Colorimetric Cyto X™ Cell Viability Assay Kit (LPS solution, Daejeon, Korea) was used. After 24 and 48 hours of incubation, the reaction solution contained in the experimental kit was mixed with each test group, followed by reaction at 37 °C in a CO₂ incubator for 2 hours. The absorbance was then measured at a wavelength of 450 nm.

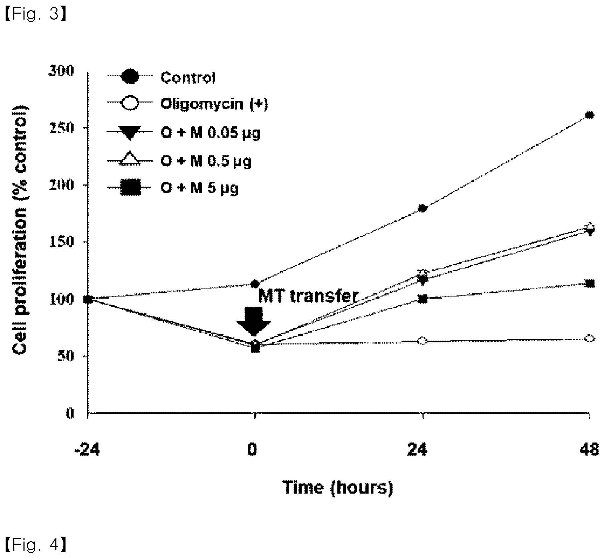

As shown in Fig. 3, it was confirmed that the absorbance values of the cell groups having received mitochondria were increased compared with the mitochondrial function-suppressed cell group. At this time, the increase of the absorbance means that the activity of mitochondrial dehydrogenase was increased while the number of viable cells of the sample increased, and thus it was confirmed that the cell proliferative capacity was enhanced through mitochondrial transfer by centrifugation.

### Example 4. Evaluation of ATP synthesis capacity of cells having received mitochondria

In the same manner as in Example 3, the cells received exogenous mitochondria after impairing mitochondrial function by the ATP synthesis inhibitor were incubated, and the resulting cells were recovered after 24 and 48 hours of incubation, respectively.

In order to measure the amount of ATP in each sample, ATP bioluminescent somatic cell assay kit (Sigma-Aldrich, St. Louis, Mo., USA) was used. ATP releasing solution was added to the prepared samples and reacted at room temperature for 20 seconds to release ATP from the sample. Then, ATP assay mix solution was added to the sample, followed by reaction at room temperature for 10 minutes, and the amount of ATP was measured using a luminometer. The amount of ATP in each sample was calculated from the ATP standard curve.

As shown in Fig. 4, when the amounts of ATP present in the samples were calculated and the changes in the amount of ATP were analyzed in comparison with the control group, it was found that the ATP synthesis capacity of the cells that received the exogenous mitochondria was higher than that of the mitochondrial function-suppressed cells.

These results indicate that when the mitochondrial function of cells is impaired, the damage is restored by transferring healthy mitochondria to the cells.

### Example 5. Evaluation of cell membrane potential after the transfer of exogenous mitochondria into muscle cells wherein muscle atrophy was induced

Actually, in order to evaluate the therapeutic effect on muscle atrophy accompanying mitochondrial dysfunction, muscle atrophy was induced in L6, a rat skeletal muscle-derived cell line, using an atrophy inducing agent (dexamethasone, Sigma-Aldrich, St. Louis, MO, USA). Subsequently, healthy mitochondria (Intact MT) extracted from WRL-68 hepatocytes and damaged mitochondria (Damaged MT) extracted from the same cells, whose mitochondrial function was impaired by treating a mitochondrial ATP synthesis inhibitor, were obtained, respectively. Mitochondria were delivered to the atrophic muscle cells at different concentrations (0.05, 0.5, and 5 µg) per 1 × 10⁵ cells, and the resulting cells were seeded into a 24-well plate and incubated at 37 °C in a CO₂ incubator.

After 48 hours of incubation, JC-1 dye was used to measure the mitochondrial membrane potential of each sample. After reacting the sample with the JC-1 dye, absorbance was measured using the property of the dye having different spectra depending on the change of membrane potential.

The dye exists as a monomer at a low concentration and exhibits green fluorescence. At a high concentration, the dye is J-aggregated to exhibit red fluorescence. Therefore, the membrane potential of the mitochondria was analyzed by calculating the ratio of green absorbance to red absorbance.

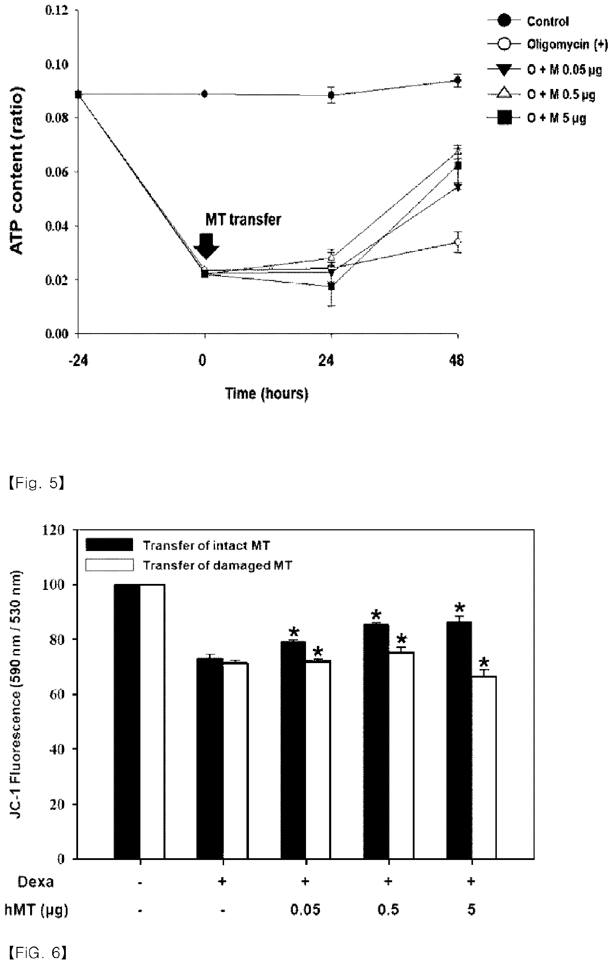

As shown in Fig. 5, it was confirmed that the membrane potential of the cells that received the intact exogenous mitochondria was recovered by about 12 to 22% as compared with the cells where the atrophy was induced and the membrane potential of the mitochondria was changed. Especially, this effect was significant when compared with the group received the damaged mitochondria.

These results imply activation of cells through the transfer of exogenous mitochondria. Therefore, it was confirmed that ATP synthesis capacity of cells can be increased by receiving exogenous mitochondria.

### Example 6. Evaluation of mitochondrial reactive oxygen species after the transfer of exogenous mitochondria into muscle cells wherein muscle atrophy was induced

In order to evaluate the reactive oxygen species in mitochondria within the muscle cells wherein muscle atrophy was induced, the respective mitochondria were transferred into the atrophic muscle cells in the same manner as in Example 5, and reactive oxygen species was evaluated after 48 hours of incubation.

MitoSOX red dye (Invitrogen, Carlsbad, CA, USA) was used for analyzing mitochondria-derived reactive oxygen species. The cells were washed with a buffer solution, and MitoSOX red dye was mixed with the medium and added to the cells. The mixture was reacted at 37 °C in a CO₂ incubator for 20 minutes. After the reaction, the cells were washed with a buffer solution, and the cover slides in the wells, in which the cells were inoculated, were collected, placed on a slide, and observed under a fluorescence microscope. Red fluorescence signal increases when reactive oxygen species in mitochondria increase due to damage. Fluorescence density of each sample was analyzed by an analysis program.

As shown in Fig. 6, it was confirmed that the reactive oxygen species were significantly reduced in the cells received intact mitochondria as compared with the cells in which muscle atrophy was induced by the method of the present invention and thus the mitochondrial reactive oxygen species were increased. Especially, this effect was significant when compared with the group received the damaged mitochondria.

It was confirmed from these results that the transfer of exogenous mitochondria affects the balance of ATP synthesis and reactive oxygen species production in the mitochondria of the cells.

### Example 7. Evaluation of biosynthesis capacity of mitochondria after the transfer of exogenous mitochondria into muscle cells wherein muscle atrophy was induced

The expression levels of PGC-1 (Peroxisome proliferator-activated receptor Gamma Coactivator-1) in the proteins were measured to evaluate the biosynthesis capacity of intracellular mitochondria. To this end, the respective mitochondria were transferred into the atrophic muscle cells in the same manner as in Example 5. After 48 hours of incubation, the resulting cells were harvested and proteins were extracted. The change in the extracted proteins of each sample was analyzed by Western blot using a PGC-1 antibody (Santa Cruz Biotechnology Inc., Santa Cruz, CA, Cat no: sc-13067).

At the normal level of mitochondrial function, mitochondrial biosynthesis is activated and PGC-1 expression is high. However, the expression of PGC-1 is lowered at the impaired level of mitochondrial function, which means mitochondrial biosynthesis is reduced.

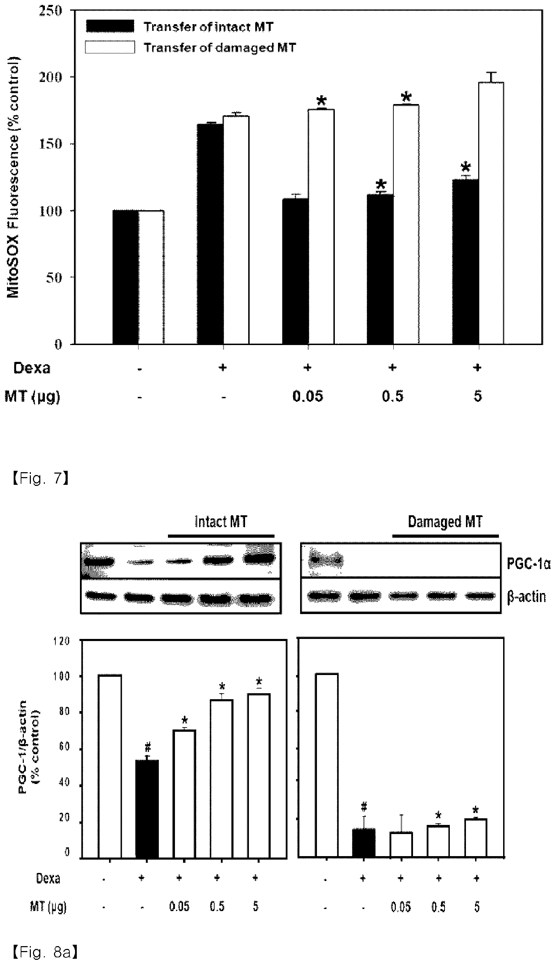

As shown in Fig. 7, it was confirmed that mitochondrial biosynthesis capacity of the cells received the intact exogenous mitochondria was improved to a level similar to that of normal cells, as compared with the cells in which mitochondria biosynthesis capacity was lowered by the induction of atrophy. Especially, this effect was significant when compared with the group received the damaged mitochondria.

From these results, it was confirmed that mitochondrial biosynthesis can be activated through the transfer of exogenous mitochondria, thereby improving the functions of intracellular mitochondria.

### Example 8. Evaluation of AMPK activation and FoxO accumulation in cells after the transfer of exogenous mitochondria into muscle cells wherein muscle atrophy was induced

The levels of AMPK activation and FoxO accumulation in the proteins were measured to compare the levels depending on change in cellular mitochondrial function. To this end, the respective mitochondria were transferred into the atrophic muscle cells in the same manner as in Example 5. After 48 hours of incubation, the resulting cells were harvested and proteins were extracted. The changes in the extracted proteins of each sample were analyzed by Western blot using AMPKα antibody (Cell Signaling Technology, Beverly, MA, #2535), Phospho-AMPKa antibody (Cell Signaling Technology, Beverly, MA, #2532), and FoxO3α antibody (Cell Signaling Technology, Beverly, MA, #2497).

When the mitochondrial function is impaired, the amount of ATP in the cell becomes insufficient. As a result, AMPK (Adeno Mono Phosphate Kinase), which is a sensor of intracellular energy metabolism, is activated. FoxO activated by this signal is accumulated in the nucleus, thereby promoting the expression of atrogenes that have influences on atrophy of muscle tissue, such as MuRF-1 and MAFbx, and consequently, leading to muscle atrophy.

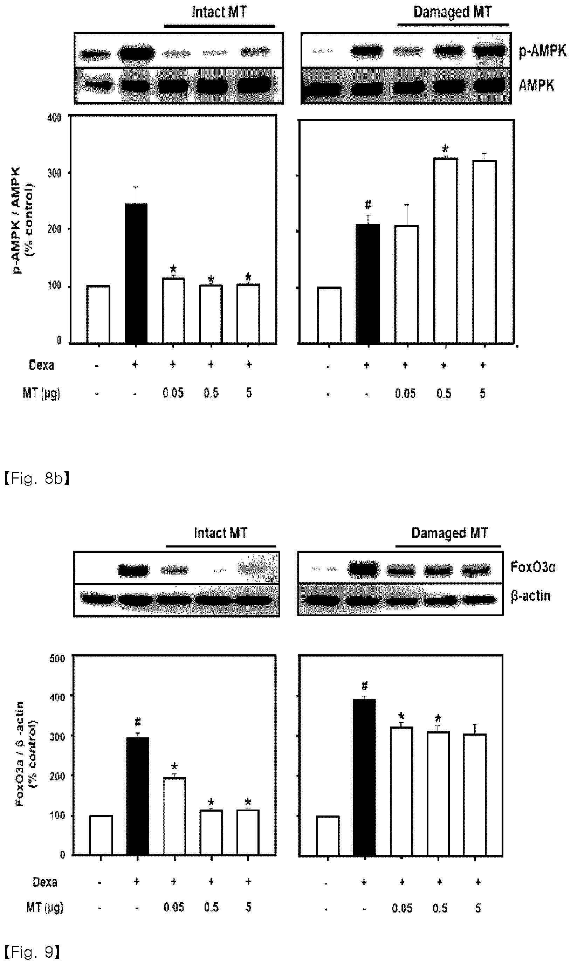

As shown in Figs. 8a and 8b, it was confirmed that the levels of AMPK activation and FoxO accumulation of the cells received intact exogenous mitochondria were reduced to levels similar to those of normal cells, as compared with the mitochondrial function-suppressed cells. Especially, this effect was significant when compared with the group received the damaged mitochondria.

From these results, it was confirmed that the signal transduction mechanism of muscle atrophy can be inhibited by suppressing the signal transduction of AMPK, which is an intracellular energy sensor, through the transfer of exogenous mitochondria and inhibiting FoxO activation thereby.

### Example 9. Evaluation of expression level of MuRF-1, a muscle atrophy marker, after the transfer of exogenous mitochondria into muscle cells wherein muscle atrophy was induced

The expression levels of MuRF-1 (muscle ring finger-1) in the proteins were measured to compare the protective efficacies against muscle atrophy by the intracellular transfer of exogenous mitochondria. The respective mitochondria were transferred into the atrophic muscle cells in the same manner as in Example 5. After 48 hours of incubation, cells were harvested and proteins were extracted. The change in the extracted proteins of each sample was analyzed by Western blot using MuRF-1 antibody (Santa Cruz Biotechnology Inc., Santa Cruz, CA, Cat no: sc-27642).

Upon induction of muscle atrophy, mitochondrial function is impaired and, accordingly, muscle atrophy transcription factors (e.g., MuRF-1) are activated by FoxO accumulated in the nucleus, as shown in Fig. 8b, thereby inducing muscle atrophy.

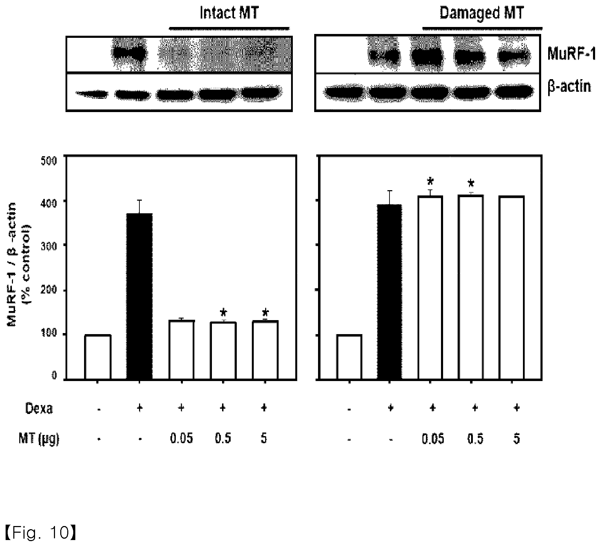

As shown in Fig. 9, the expression level of MuRF-1 was reduced to a level similar to that of normal cells in the group to which intact exogenous mitochondria were transferred, as compared with the increase in the expression level of MuRF-1 in the muscle atrophy induction group. Especially, this effect was significant when compared with the group received the damaged mitochondria.

These results are consistent with the theory that muscle atrophy can be induced by the stimulation of AMPK-FoxO/Atrogene pathway due to mitochondrial dysfunction accompanying the induction of muscle atrophy. In fact, it has been confirmed through the present invention that muscle atrophy can be treated by externally delivering healthy mitochondria and thereby blocking this pathway.

### Example 10. Measurement of weight change of the soleus muscle by mitochondrial transplantation

The mitochondria isolated in Example 2 were assayed for mitochondrial protein levels through BCA assay, and the administration doses were set at a low concentration (MT low; 0.5 µg) and a high concentration (MT high; 5 µg). These concentrations were within the effective concentration range for which the recovery effect was verified in the cells wherein muscle atrophy had been induced. The prepared mitochondria were suspended in 100 µl of saline, put into a 0.25 mm (31G) × 8 mm insulin syringe (BD Ultra-Fine II), and injected up and down twice into the gastrocnemius muscle of the muscle atrophy model rats prepared in Example 1. To the muscle atrophy induction group, 0.9 % (v/v) saline of the same volume was injected.

Autopsies were performed on days 1, 7, and 14 after the mitochondrial transplantation. The body weight and the weight of the soleus muscle were measured for each experimental group. Based on the measured weights, changes in the weight of soleus muscle relative to the body weight were analyzed.

As a result, when the saline was administered to the rats wherein muscle atrophy was induced, the weight of the soleus muscle was reduced to about twice that of the normal control group. It was confirmed that the weight change was restored from day 1 after mitochondrial transplantation and the weight of the soleus muscle was increased depending on the concentration of delivered mitochondria.

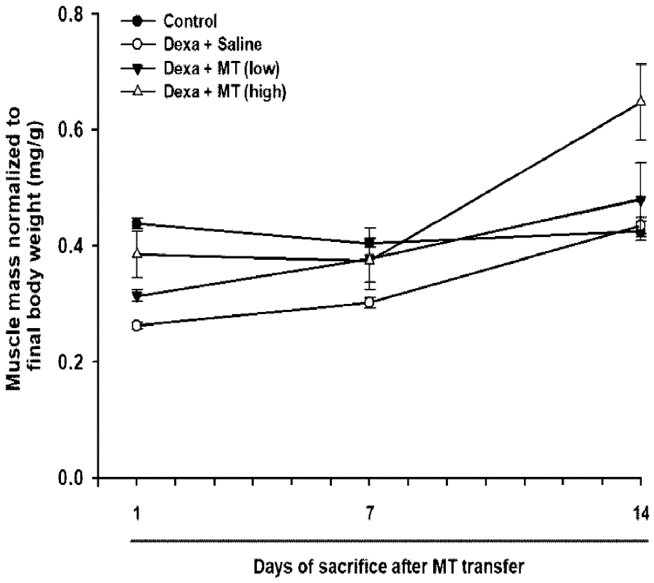

On day 14 after the dexamethasone treatment, it was confirmed that the muscle atrophy induction group also recovered naturally. In addition, it was confirmed that the weight of the soleus muscle was increased by the mitochondrial transplantation than the normal control group (Fig. 10).

### Example 11. Confirmation of the expression of mitochondrial biosynthetic protein by mitochondrial transplantation

In order to compare the mitochondrial biosynthesis activities in the soleus muscles collected on days 1, 7 and 14 after mitochondrial transplantation, the expression levels of PGC-1 were confirmed by Western blot.

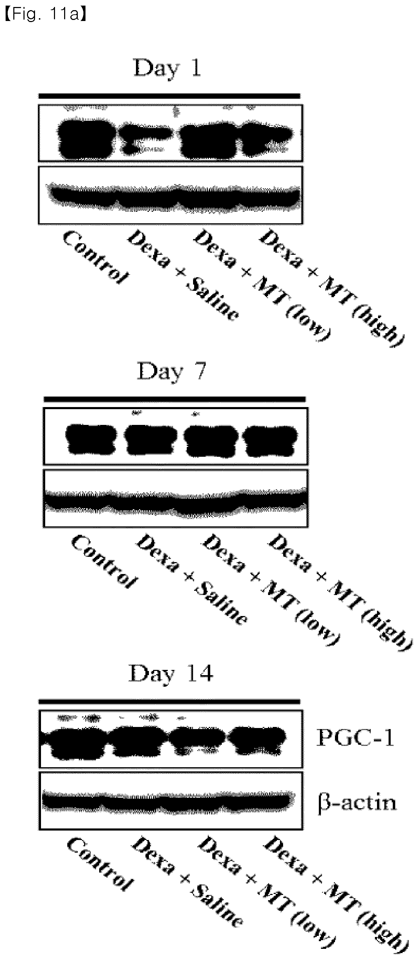

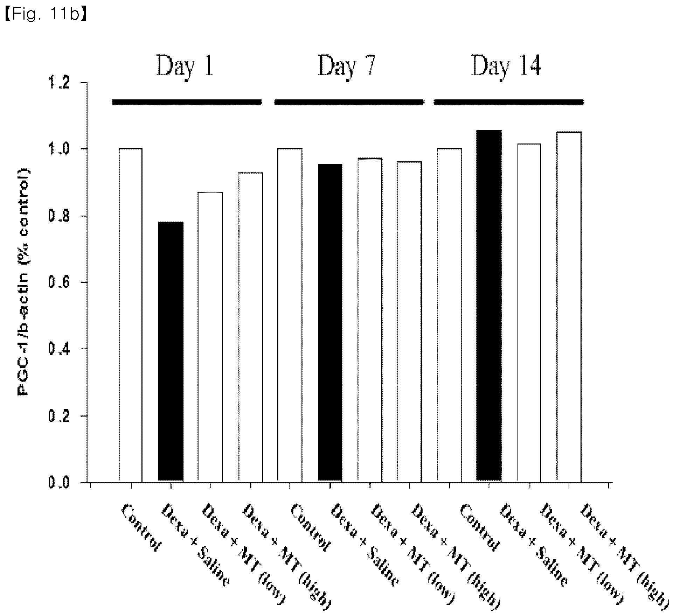

On day 1 after the induction of muscle atrophy, the amount of PGC-1 expression decreased due to muscle atrophy. To the contrary, PGC-1 expression was restored in the mitochondrial transplantation groups depending on the concentration of transplanted mitochondria. On the other hand, it was confirmed that the expression levels of PGC-1 were restored similarly to the normal group in the muscle atrophy induction group on 7 and 14 days in the natural recovery stage (Figs. 11a and 11b).

In other words, it was confirmed that the mitochondrial biosynthesis was inhibited by the induction of muscle atrophy, and that the expression amount of PGC-1 in mitochondria in tissues was increased after exogenous mitochondrial transplantation. Therefore, it was confirmed that transplantation of exogenous mitochondria could increase the activity of mitochondrial biosynthesis.

### Example 12. Expression of muscle atrophy-related protein by mitochondrial transplantation

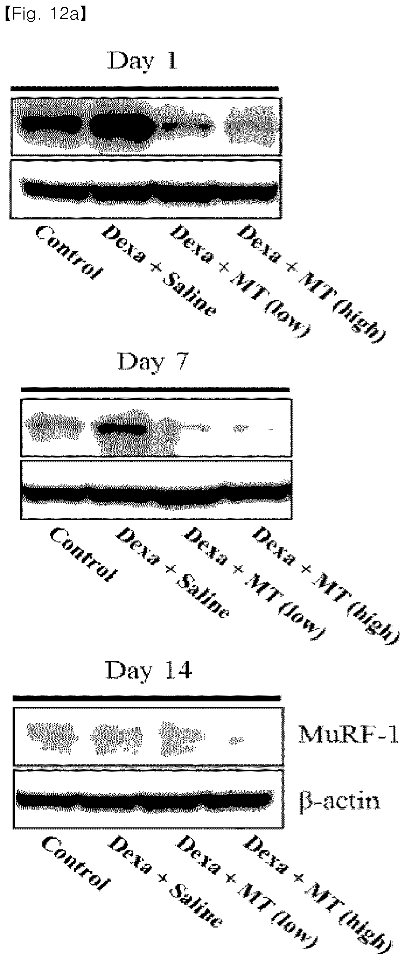

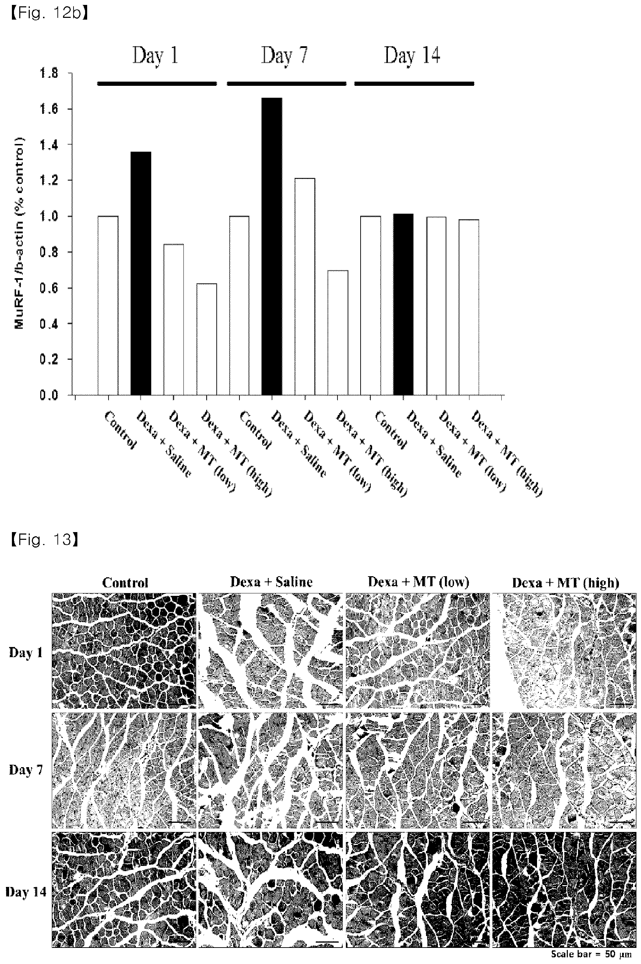

The expression levels of MuRF-1, a specific marker of muscle atrophy, were confirmed by Western blot in the soleus muscles collected on days 1, 7, and 14 after mitochondrial transplantation. It was confirmed that, on day 7, MuRF-1 expression level was increased by the induction of muscle atrophy. On the other hand, it was confirmed that the expression of MuRF-1 was inhibited in the groups received mitochondria depending on the concentration of transplanted mitochondria. In addition, no expression of MuRF-1 in the muscle atrophy induction group on day 14 indicates a time point of natural recovery from the muscle atrophy (Figs. 12a and 12b).

In other words, the expression of MuRF-1 was confirmed in the muscles wherein muscle atrophy was induced, and the expression continued until day 7. On the other hand, it was confirmed that the expression of MuRF-1 was suppressed after the transplantation of exogenous mitochondria. Thus, it was confirmed from these results that it is possible to treat muscle atrophy by transplanting exogenous mitochondria.

### Example 13. Histological analysis of muscle fiber regeneration by mitochondrial transplantation

After inducing muscle atrophy, exogenous mitochondria were transplanted and their effect of treating the muscle atrophy was analyzed histologically. As a result of H & E staining, it was confirmed that the size of myofiber decreased and the endomysium spread widely in the muscle atrophy induction group, as compared with the normal control group, until day 7 after the transplantation. On the other hand, in the group transplanted with exogenous mitochondria, the size of muscle fibers increased and density thereof was high. On day 14, it was confirmed that the muscle atrophy induction group showed a natural recovery in that the size of the myofiber and the endomysium became similar to the normal control group (Fig. 13).

From these results, it was confirmed that muscle regeneration can be promoted by transplanting exogenous mitochondria into the muscle impaired by the induction of muscle atrophy.

### II. Confirmation of the effects of treating an ischemic disease of mitochondria and cells containing exogenous mitochondria

### Comparative Example. Preparation of a pharmaceutical composition comprising placenta-derived mesenchymal stem cells

The mesenchymal stem cells derived from the placenta (Provided by CHA Bundang Medical Center, IRB No. 1044308-201511-BR-022-02) were suspended in Alpha-MEM (Alpha-Minimum Essential Medium) containing 10 % (v/v) fetal bovine serum (FBS, Gibco), 100 µg/ml of streptomycin, and 100 U/ml of ampicillin, and cultured for 72 hours. After the incubation was completed, the cells were washed twice with DPBS (Dulbecco's phosphate buffered saline, Gibco). The washed cells were treated with 0.25% trypsin-EDTA (TE, Gibco) to obtain cells.

The cells thus obtained were washed twice with DPBS, mixed with 100 µl of water for injection in amounts of 1 × 10⁵, 1 × 10⁶, and 5 × 10⁶ cells, respectively, and filled in insulin syringes.

### Example 14. Preparation of a pharmaceutical composition comprising mitochondria

The mesenchymal stem cells derived from the placenta were suspended in Alpha-MEM containing 10 % (v/v) fetal bovine serum, 100 µg/ml of streptomycin, and 100 U/ml of ampicillin, and cultured for 72 hours. After the incubation was completed, the cells were washed twice with DPBS. The washed cells were treated with 0.25% trypsin-EDTA to obtain cells.

In order to extract mitochondria from the obtained cells, the number of cells was counted using a hemocytometer to collect cells of about 3 × 10⁶ cells/ml. The cells were then subjected to the first centrifugation at a temperature of about 4 °C for 10 minutes at a speed of 350 x g, and the resulting pellet was recovered, resuspended in a buffer solution, and homogenized for 10 to 15 minutes. The homogenate thus obtained was subjected to the second centrifugation at a temperature of about 4 °C for 3 minutes at a speed of 1,100 x g to obtain a supernatant. The supernatant was then subjected to the third centrifugation at a temperature of about 4 °C for 15 minutes at a speed of 12,000 x g to isolate the mitochondria from the cells.

The mitochondria thus obtained were mixed with 100 µl of water for injection in amounts of 0.2, 2, and 10 µg, respectively, and filled in insulin syringes.

### Example 15. Preparation of a pharmaceutical composition comprising cells to which mitochondria were transferred

The mitochondria isolated from the placenta-derived mesenchymal stem cells (donor cells) according to the method of Example 14 were put into three test tubes containing the mesenchymal stem cells (target cells) derived from different placenta in amounts of 1 x 10⁵, 1 x 10⁶, and 5 x 10⁶ cells, respectively, and the mixtures were centrifuged at a temperature of about 4 °C for 15 minutes at a speed of 1,500 x g. The supernatant was removed, and the cells were washed with PBS and centrifuged at a temperature of about 4 °C for 5 minutes. Washing was performed twice under the same conditions. At this time, the isolated mitochondria were delivered to the target cells at a weight of 2 µg per 1 × 10⁵, 1 × 10⁶, and 5 × 10⁶ cells, respectively.

The thus obtained cells, to which mitochondria were delivered, were mixed with 100 µl of water for injection in amounts of 1 × 10⁵, 1 × 10⁶, and 5 × 10⁶ cells, respectively, and filled in insulin syringes.

### Example 16. Confirmation of the transfer of exogenous mitochondria

In order to confirm whether the exogenous mitochondria were transferred to cells in Example 15, for the purpose of distinguishing between the mitochondria of donor cells and the mitochondria of target cells, the mitochondria of donor cells were labeled with green fluorescence (Mitotracker green) and the mitochondria of the target cells were labeled with red fluorescence (Mitotracker red).

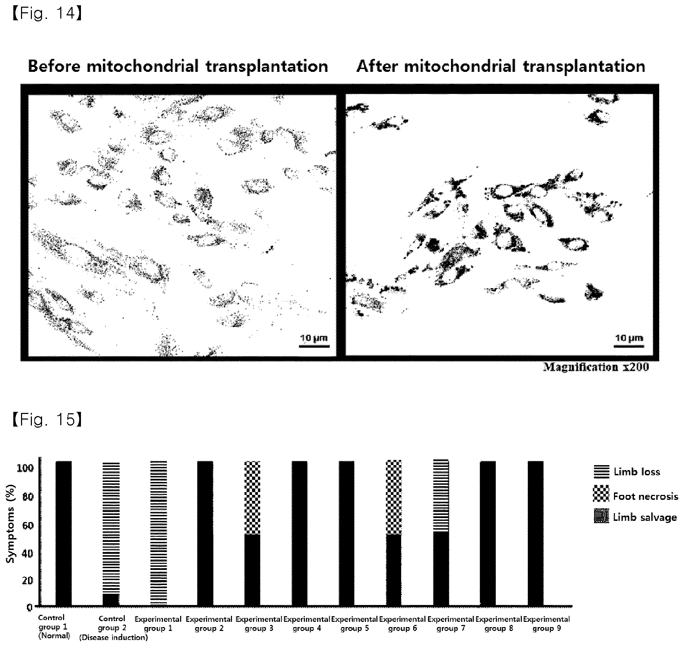

Fluorescence-labeled cells were fixed on slides using a fixative containing DAPI for staining nuclei and photographed using a confocal scanning microscope (Zeiss LSM 880 Confocal microscope), the photographs being shown in Fig. 14.

As shown in Fig. 14, a yellowish part was observed in the cells having received mitochondria. This part was formed through the merged luminescent colors due to the co-localization of the target cell mitochondria emitting red light and the donor cell mitochondria luminescing green light, and it was confirmed from such result that the mitochondria of donor cells were transferred to the target cells.

### Example 17. Evaluation of treatment for critical limb ischemia through visual inspection

Five to six-week-old male Balb/c nude mice were purchased from Orient Bio Co., Ltd. (Seoul, Korea). The mice were acclimatized in the clean zone of the Experimental Animal Center of CHA University. During the acclimation period, the environment of the mice was maintained at a 12-h light/dark cycle, a room temperature of 23 ± 2 °C, and a humidity of 40 to 60 %. After 7 days of such acclimation period, the mice were subjected to the experiments.

The arterial blood vessels of the lower limbs of the mice thus prepared were ligated by surgical operation to induce critical lime ischemia. At this time, 100 µl each of the pharmaceutical compositions according to Comparative Example, Example 14, and Example 15 were administered by intramuscular injection (IM) to the thigh region, where the surgical operation was performed, to prepare Experimental groups 1 to 9 (See Table 1 below).

**[Table 1]**

| | Exp. Group 1 | Exp. Group 2 | Exp. Group 3 | Exp. Group 4 | Exp. Group 5 | Exp. Group 6 | Exp. Group 7 | Exp. Group 8 | Exp. Group 9 |
|---|---|---|---|---|---|---|---|---|---|
| Administered Composition | Compar. Ex. (MSC) | Compar. Ex. (MSC) | Compar. Ex. (MSC) | Ex. 14 (MT) | Ex. 14 (MT) | Ex. 14 (MT) | Ex. 15 (2 µg MT transferred MSC) | Ex. 15 (2 µg MT transferred MSC) | Ex. 15 (2 µg MT transferred MSC) |
| Dose | 1 x 10⁵ | 1 x 10⁶ | 5 x 10⁶ | 0.2 µg | 2 µg | 10 µg | 1 x 10⁵ | 1 x 10⁶ | 5 x 10⁶ |

On the other hand, a normal control group (Control group 1) was prepared with normal mice not received injections or surgical operations. In addition, Control group 2 was prepared in the same manner as Experimental group 1, except that 100 µl of water for injection was administered by intramuscular injection (IM).

After 7 days, in order to evaluate the symptoms visually, the effects of treatment were evaluated by dividing into limb loss, foot necrosis, and limb salvage for Experimental groups 1 to 9 and Control groups 1 and 2, and the results are shown in Fig. 15.

As shown in Fig. 15, no disease occurred in the normal mice of Control group 1, but the mice of Control group 2 to which physiological saline was administered underwent more than 90% of the limb injury. In addition, in the mice of Experimental groups 1, 2, and 3 to which placenta-derived mesenchymal stem cells were administered, it was found that not less than 40% of limb salvage and not less than 50 % of limb loss occurred. On the contrary, mice of Experimental groups 4, 5, and 6 to which mitochondria were administered showed 60 to 100% of limb salvage and less than 20% of limb loss. In addition, in the mice administered with mitochondria-transferred cells, no limb loss were observed in the mice of Experimental groups 8 and 9, and only 50% of the limb loss occurred in the mice of Experimental group 7.

From the above results, it was confirmed that administration of the pharmaceutical compositions containing mitochondria or mitochondria-transferred cells according to the present invention has the effect of improving and treating critical limb ischemia symptoms. In particular, it was confirmed that the therapeutic effect was superior to that of the general cases where only the isolated stem cells were administered.

### Example 18. Evaluation of treatment for critical limb ischemia through measurement of blood flow

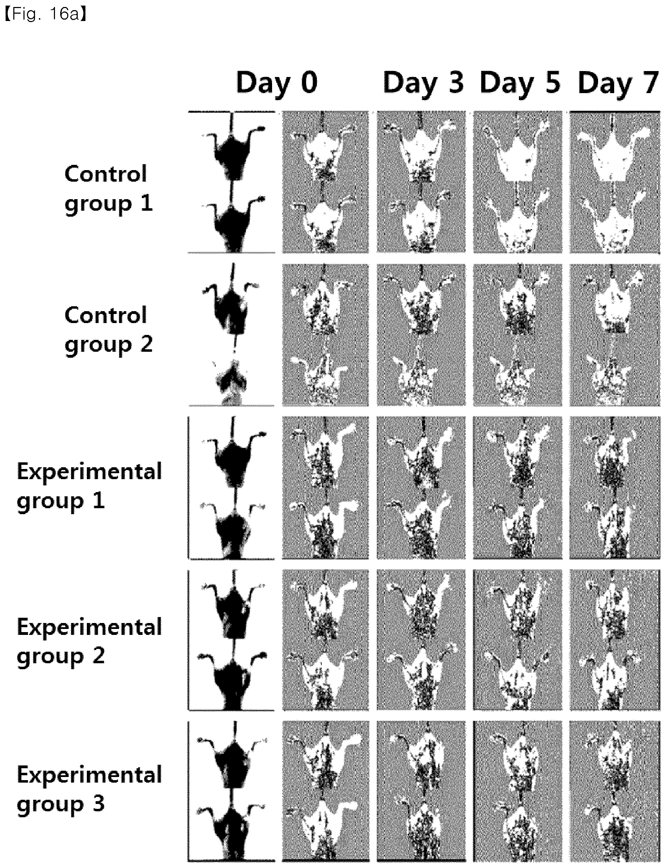

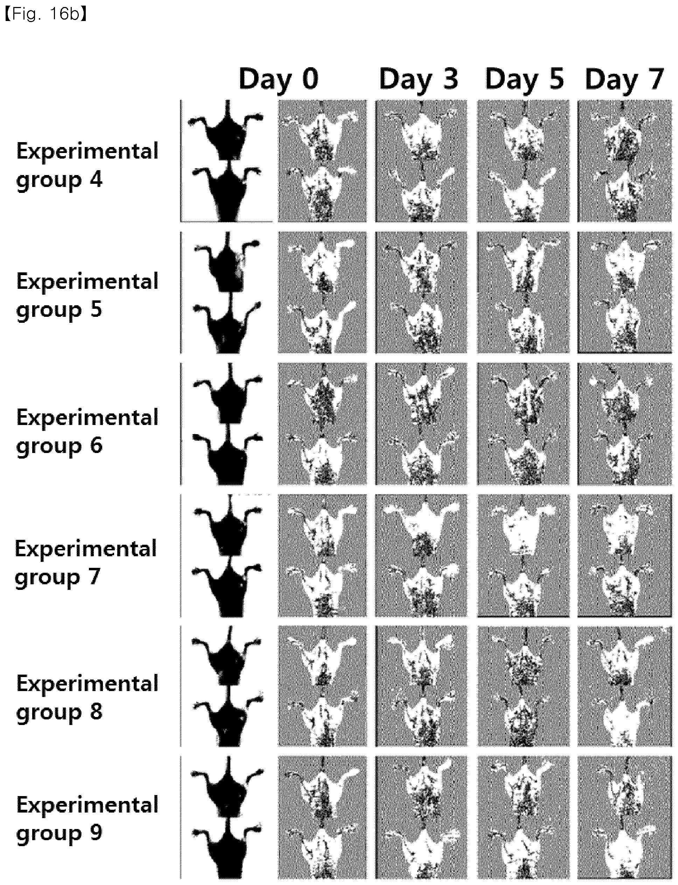

In order to evaluate the blood flow of lower extremity for Experimental groups 1 to 9 and Control groups 1 and 2, the blood flow at certain time points (immediately after administration, and 3, 5, 7, and 14 days after administration) was measured via laser Doppler imaging (LDI) and the results are shown in Figs. 16a and 16b.

As shown in Fig. 16a, it was confirmed that the blood flow was measured in red and green in the normal mice of Control group 1, but the blood flow was not measured in the mice of Control group 2 administered with physiological saline due to the limb loss. In addition, it was confirmed that in Experimental groups 1 to 3 in which the pharmaceutical composition containing the placenta-derived stem cells alone (Comparative example) was administered, the blood flow was not measured similarly to Control group 2.

Alternatively, as shown in Fig. 16b, it was confirmed that in Experimental groups 4 and 6, which were administered with the pharmaceutical composition containing mitochondria (Example 14), and Experimental groups 8 and 9, which were administered with the pharmaceutical composition containing cells received mitochondria (Example 15), blood flow was measured at levels similar to that of the normal mice of Control group 1. In addition, it was confirmed that the blood flow in Experimental group 5, to which mitochondria were administered, and Experimental group 7, to which the cells received mitochondria were administered, was measured at levels to be about 50% in comparison with Control group 1.

While the present invention has been described by way of examples, those skilled in the art will appreciate that various changes and modifications may be made in the present invention by additions, modifications, deletions, supplements, or the like of components, without departing from the scope of the present invention as set forth in the appended claims, and such changes and modifications are also included within the scope of the present invention.

## Claims

1. A pharmaceutical composition for preventing or treating a muscle disease, comprising mitochondria as an active ingredient.

2. The pharmaceutical composition for preventing or treating a muscle disease of claim 1, wherein the mitochondria are those isolated from cells or tissues.

3. The pharmaceutical composition for preventing or treating a muscle disease of claim 2, wherein the cells are any one selected from the group consisting of somatic cells, germ cells, stem cells, and combinations thereof.

4. The pharmaceutical composition for preventing or treating a muscle disease of claim 3, wherein the somatic cells are any one selected from the group consisting of muscle cells, hepatocytes, neurons, fibroblasts, epithelial cells, adipocytes, bone cells, leukocytes, lymphocytes, platelets, mucosal cells, and combinations thereof.

5. The pharmaceutical composition for preventing or treating a muscle disease of claim 3, wherein the germ cells are any one selected from the group consisting of sperms, eggs, and combinations thereof.

6. The pharmaceutical composition for preventing or treating a muscle disease of claim 3, wherein the stem cells are any one selected from the group consisting of mesenchymal stem cells, adult stem cells, induced pluripotent stem cells, embryonic stem cells, bone marrow stem cells, neural stem cells, limbal stem cells, tissue-derived stem cells, and combinations thereof.

7. The pharmaceutical composition for preventing or treating a muscle disease of claim 6, wherein the mesenchymal stem cells are obtained from any one selected from the group consisting of umbilical cord, cord blood, bone marrow, fat, muscle, nerve, skin, amniotic membrane, placenta, and combinations thereof.

8. The pharmaceutical composition for preventing or treating a muscle disease of claim 1, which comprises the mitochondria at a concentration of 0.1 to 500 µg/ml.

9. The pharmaceutical composition for preventing or treating a muscle disease of claim 1, wherein the muscle disease is MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, or myotonia.

10. A pharmaceutical composition for preventing or treating a muscle disease, comprising cells into which exogenous mitochondria are introduced, as an active ingredient.

11. The pharmaceutical composition for preventing or treating a muscle disease of claim 10, wherein the cells are muscle cells or stem cells.

12. The pharmaceutical composition for preventing or treating a muscle disease of claim 11, wherein the stem cells are any one selected from the group consisting of mesenchymal stem cells, adult stem cells, induced pluripotent stem cells, embryonic stem cells, bone marrow stem cells, neural stem cells, limbal stem cells, tissue-derived stem cells, and combinations thereof.

13. The pharmaceutical composition for preventing or treating a muscle disease of claim 12, wherein the mesenchymal stem cells are obtained from any one selected from the group consisting of umbilical cord, cord blood, bone marrow, fat, muscle, nerve, skin, amniotic membrane, placenta, and combinations thereof.

14. The pharmaceutical composition for preventing or treating a muscle disease of claim 10, wherein the exogenous mitochondria are transferred to the cells by centrifuging a mixture of the cells and the exogenous mitochondria.

15. The pharmaceutical composition for preventing or treating a muscle disease of claim 10, which comprises the cells into which 0.1 to 500 µg of the mitochondria are introduced per 1 x 10⁵ cells.

16. The pharmaceutical composition for preventing or treating a muscle disease of claim 10, wherein the muscle disease is MELAS syndrome, MERRF syndrome, Kearns-Sayre syndrome, myopathy, encephalomyopathy, myasthenia, myasthenia gravis, amyotrophic lateral sclerosis, muscular dystrophy, muscular atrophy, or myotonia.

17. A method of preventing or treating a muscle disease, which comprises administering the pharmaceutical composition according to any one of claims 1 to 16 to the affected part of a subject in need thereof.

18. A pharmaceutical composition for preventing or treating an ischemic disease, comprising mitochondria as an active ingredient.

19. The pharmaceutical composition for preventing or treating an ischemic disease of claim 18, wherein the mitochondria are isolated from cells or tissues.

20. The pharmaceutical composition for preventing or treating an ischemic disease of claim 19, wherein the cells are any one selected from the group consisting of somatic cells, germ cells, stem cells, and combinations thereof.

21. The pharmaceutical composition for preventing or treating a muscle disease of claim 20, wherein the somatic cells are any one selected from the group consisting of muscle cells, hepatocytes, neurons, fibroblasts, epithelial cells, adipocytes, bone cells, leukocytes, lymphocytes, platelets, mucosal cells, and combinations thereof.

22. The pharmaceutical composition for preventing or treating a muscle disease of claim 20, wherein the germ cells are any one selected from the group consisting of sperms, eggs, and combinations thereof.

23. The pharmaceutical composition for preventing or treating a muscle disease of claim 20, wherein the stem cells are any one selected from the group consisting of mesenchymal stem cells, adult stem cells, induced pluripotent stem cells, embryonic stem cells, bone marrow stem cells, neural stem cells, limbal stem cells, tissue-derived stem cells, and combinations thereof.

24. The pharmaceutical composition for preventing or treating a muscle disease of claim 23, wherein the mesenchymal stem cells are obtained from any one selected from the group consisting of umbilical cord, cord blood, bone marrow, fat, muscle, nerve, skin, amniotic membrane, placenta, and a combination thereof.

25. The pharmaceutical composition for preventing or treating an ischemic disease of claim 18, which comprises the mitochondria at a concentration of 0.1 to 500 µg/ml.

26. The pharmaceutical composition for preventing or treating an ischemic disease of claim 18, wherein the ischemic disease is critical limb ischemia, ischemic stroke, ischemic heart disease, or ischemic colitis.

27. A pharmaceutical composition for preventing or treating an ischemic disease, comprising cells into which exogenous mitochondria are introduced, as an active ingredient.

28. The pharmaceutical composition for preventing or treating an ischemic disease of claim 27, wherein the cells are muscle cells or stem cells.

29. The pharmaceutical composition for preventing or treating a muscle disease of claim 28, wherein the stem cells are any one selected from the group consisting of mesenchymal stem cells, adult stem cells, induced pluripotent stem cells, embryonic stem cells, bone marrow stem cells, neural stem cells, limbal stem cells, tissue-derived stem cells, and combinations thereof.

30. The pharmaceutical composition for preventing or treating a muscle disease of claim 29, wherein the mesenchymal stem cells are obtained from any one selected from the group consisting of umbilical cord, cord blood, bone marrow, fat, muscle, nerve, skin, amniotic membrane, placenta, and combinations thereof.

31. The pharmaceutical composition for preventing or treating an ischemic disease of claim 27, wherein the exogenous mitochondria are transferred to the cells by centrifuging a mixture of the cells and the exogenous mitochondria.

32. The pharmaceutical composition for preventing or treating an ischemic disease of claim 27, which comprises the cells into which 0.1 to 500 µg of the mitochondria are introduced per 1 x 10⁵ cells.

33. The pharmaceutical composition for preventing or treating an ischemic disease of claim 27, wherein the ischemic disease is critical limb ischemia, ischemic stroke, ischemic heart disease, or ischemic colitis.

34. A method of preventing or treating an ischemic disease, which comprises administering the pharmaceutical composition according to any one of claims 18 to 33 to the affected part of a subject in need thereof.

35. A use of the pharmaceutical composition of claim 1 or 10 for preventing or treating a muscle disease.

36. A use of the pharmaceutical composition of claim 1 or 10 for the manufacture of a medicament for preventing or treating a muscle disease.

37. A use of the pharmaceutical composition of claim 18 or 27 for preventing or treating an ischemic disease.

38. A use of the pharmaceutical composition of claim 18 or 27 for the manufacture of a medicament for preventing or treating an ischemic disease.
